(19) Europäisches Patentamt
European Patent Office
Office européen des brevets

(11) EP 4 813 393 A1

(12) **EUROPEAN PATENT APPLICATION**
published in accordance with Art. 153(4) EPC

(43) Date of publication:
**30.09.2026 Bulletin 2026/40**

(21) Application number: **24894065.2**

(22) Date of filing: **14.11.2024**

(51) International Patent Classification (IPC):
***A61K 31/519*** *(2006.01)* ***A61K 9/1271*** *(2025.01)*
***A61K 47/10*** *(2017.01)* ***A61K 47/24*** *(2006.01)*
***A61P 25/00*** *(2006.01)* ***A61P 43/00*** *(2006.01)*

(52) Cooperative Patent Classification (CPC):
**A61K 9/1271; A61K 31/519; A61K 47/10; A61K 47/24; A61P 25/00; A61P 43/00**

(86) International application number:
**PCT/JP2024/040391**

(87) International publication number:
**WO 2025/110074 (30.05.2025 Gazette 2025/22)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **22.11.2023 JP 2023198015**

(71) Applicant: **Chemiteras, Inc.**
**Yokohama-shi, Kanagawa 222-0033 (JP)**

(72) Inventor: **FALKENBERG, Torkei**
**DECEASED (ZZ)**

(74) Representative: **Kraus & Lederer PartGmbB**
**Thomas-Wimmer-Ring 15**
**80539 München (DE)**

(54) **LIPOSOME PREPARATION OF DEAZAFLAVIN**

(57) A technique for formulating a deazaflavin using a liposome preparation characterized by containing a deazaflavin and a polyethylene glycol-modified phospholipid is provided.

FIG.1

Before Nano
After Nano Day-0
After Nano Day-5, 2-8°C
After Nano Day-7, 2-8°C
After Nano Day-15, 2-8°C
After Nano Day-5, 25°C
After Nano Day-7, 25°C
After Nano Day-15, 25°C

EP 4 813 393 A1

## Description

### Technical Field

The present invention relates to a liposome preparation of a deazaflavin.

### Background Art

Deazaflavin (5-deazaflavin) is a generic term for a group of compounds which have a structure in which the nitrogen at the 5th position of the flavin skeleton is replaced with carbon and which have various chemical properties, for example, by being involved in various metabolic reactions, activation of oxygen and chemiluminescence as a coenzyme in the body.

The biokinetics and the chemical properties of deazaflavins are similar to those of NAD (nicotinamide·adenine·dinucleotide)$^+$, FAD (flavin adenine dinucleotide), and deazaflavins activate energy production (ATP) in the cells. Accordingly, deazaflavins are members which cause unusual properties of having a very wide variety of efficacies.

The present inventor and the like have found a deazaflavin which is excellent in activating ATP in the mitochondrial complex, of deazaflavins, and filed a patent application (PTL 1).

The deazaflavin can be possibly applied to treatment of various diseases, but the deazaflavin has a problem because the solubility is extremely low and because the deazaflavin is difficult to be formed into various generally used preparation forms.

Therefore, a technique for formulating a deazaflavin is required.

### Citation List

### Patent Literature

PTL 1: JP6717989B

### Summary of Invention

### Technical Problem

Therefore, a problem of the invention is to provide a technique for formulating a deazaflavin.

### Solution to Problem

As a result of intensive studies to solve the problem, the present inventor and the like have found that an excellent liposome preparation is obtained using a polyethylene glycol-modified phospholipid and thus have completed the invention.

That is, the invention is a liposome preparation characterized by containing a deazaflavin and a polyethylene glycol-modified phospholipid.

### Advantageous Effects of Invention

The liposome preparation of the invention is a new preparation form of a deazaflavin. Moreover, the liposome preparation is stable and has permeability through the blood-brain barrier and is thus particularly excellent for treating a brain disease.

### Brief Description of Drawings

[FIG. 1] A figure showing the results of the storage test in Test Example 1.
[FIG. 2] A figure illustrating the device used for the blood-brain barrier passing property test in Test Example 2.

### Description of Embodiments

The liposome preparation of the invention contains a deazaflavin and a polyethylene glycol-modified phospho-

lipid.

The deazaflavin which is an essential component of the liposome preparation of the invention is a component which has a structure similar to that of naturally occurring vitamin B2 and which is similar to coenzyme NAD synthesized from NMN. For the liposome preparation of the invention, any known deazaflavin can be used, but 5-deazaflavins (pyrimido[4,5-b]quinoline-2,4(3H,10H)-diones) represented by the formula (I) below are preferable. The 5-deazaflavins are described in detail in JP6717989B (PTL 1). The 5-deazaflavins are excellent in activating ATP in the mitochondrial complex.

[Chem. 1]

(I)

(In the formula, $R_1$ represents a hydrogen atom, an alkyl group, a halogen-substituted alkyl group, a carboxy-substituted alkyl group or a phenyl group, $R_2$ represents an alkyl group, a cycloalkyl group, a phenyl-substituted lower alkyl group, a phenyl group, a phenyl group substituted with one of a halogen atom, a lower alkyl group and a lower alkoxy group or a lower alkyl-disubstituted phenyl group, and $R_3$ and $R_4$ represent a hydrogen atom, a lower alkyl group, a halogen atom, a hydroxyl group, a nitro group, a cyano group, a lower alkoxy group, a phenyl-substituted lower alkoxy, a lower alkylamino group, a phenyl-substituted lower alkylamino group or a lower alkylsulfonyl group.)

Of the 5-deazaflavins, the compound named NMN-T, in which $R_1$ is a methyl group, $R_2$ is an ethyl group and $R_3$ and $R_4$ are hydrogen, is a compound managed by Chemiteras, Inc. as TND1128JP and can be acquired from Chemiteras, Inc. under the product name "5-DEAZAFLAVIN REDOX" or the like.

The polyethylene glycol-modified phospholipid which is the other essential component of the liposome preparation of the invention is not particularly limited, but examples thereof include one in which polyethylene glycol is bonded to a phospholipid that is used for a known liposome preparation.

Examples of the phospholipid include natural phospholipids such as phosphatidylcholine (PC), phosphatidylserine (PS), phosphatidylglycerol (PG), phosphatidylinositol, phosphatidylethanolamine (PE), dimyristoylphosphatidylcholine (DMPC), phosphatidic acid (PA), cardiolipin (CL), sphingomyelin (SPH), egg yolk lecithin, soy lecithin and lysolecithin, hydrogenated phospholipids obtained from natural phospholipids by a normal method such as hydrogenated soy phospholipid (HSPC) and derivatives such as DSPE (1,2 distearoyl-sn-glycerol-3-phosphatidylethanolamine). Of the phospholipids, DSPE is preferable. One kind or two or more kinds of the phospholipids can be used.

The phospholipid described above is modified with polyethylene glycol by a known method. The molecular weight of the polyethylene glycol may be appropriately selected, but, for example, the average molecular weight is 1000 to 5000, preferably 1000 to 3000 and more preferably 2000. The average molecular weight is a value measured with size exclusion chromatography (SEC) with a differential refractometer detector or a single quadrupole MS or the like.

The polyethylene glycol-modified phospholipid described above is preferably one which is represented by the formula below and in which the polylethylene glycol has the above molecular weight.

[Chem. 2] Phospholipid-polyethylene glycol

Moreover, as shown in the formulae below, the OH at the end of the polyethylene glycol is preferably a substituent such as COOH and $NH_2$.

[Chem. 3] Phospholipid-polyethylene glycol-COOH

[Chem. 4] Phospholipid-polyethylene glycol-$NH_2$

Specific examples of the polyethylene glycol-modified phospholipid described above include DSPE-$PEG_{2000}$, DSPE-$PEG_{2000}$-COOH and DSPE-$PEG_{2000}$-$NH_2$. Of these, DSPE-$PEG_{2000}$-COOH and DSPE-$PEG_{2000}$-$NH_2$ are preferable, and DSPE-$PEG_{2000}$-COOH is more preferable.

These polyethylene glycol-modified phospholipids are commercially available from various manufacturers for liposome formation. An example of DSPE-$PEG_{2000}$-COOH is 880135P of Avanti Polar Lipids (1,2-distearoyl-sn-gly-

cero-3-phosphoethanolamine-N-[carboxy(polyethylene glycol)-2000] (sodium salt)), and an example of DSPE-$PEG_{2000}$-$NH_2$ is 880128P of Avanti Polar Lipids (1,2-distearoyl-sn-glycero-3-phosphoethanolamine-N-[amino(polyethylene glycol)-2000] (ammonium salt)).

The liposome preparation of the invention can be produced by blending another component according to the need and entrapping the deazaflavin by a known liposome formation method, except for using the deazaflavin and the polyethylene glycol-modified phospholipid.

The liposome formation method is not particularly limited, but examples thereof include thin-film hydration, detergent removal method, solvent injection method and reverse-phase evaporation method. Of the methods, solvent injection method using ethanol is preferable.

Examples of the other component which is used when the liposome is formed include a solvent, a surfactant, an emollient, a stabilizer, a buffer, a solution-state stabilizer and a phospholipid.

Examples of the solvent/surfactant include water, physiological saline, benzyl alcohol, ethanol, methanol, $\alpha$-tocopherol, triolein, tricaprylin, propylene glycol and polyoxyethylene sorbitan oleate. Here, the phospholipid and the polyethylene glycol-modified phospholipid described above are sometimes used as surfactants. Examples of the buffer/solution-state stabilizer include disodium succinate, glycine, calcium chloride, sodium citrate, citric acid, sodium phosphate, sodium chloride, triethanolamine, histidine, ammonium sulfate and sucrose. An example of the emollient/-stabilizer is cholesterol. One kind of the components or a combination of two or more kinds thereof can be used.

A preferable composition for forming the liposome preparation of the invention is as shown below.

Deazaflavin: 0.0001 to 5% (w/v), preferably 0.01 to 0.10% (w/v)
Polyethylene glycol-modified phospholipid: 0.01 to 5% (w/v), preferably 0.1 to 1% (w/v)
According to the need, the following additives are contained.
Solvent/surfactant: 70 to 98% (w/v), preferably 80 to 92% (w/v)
Emollient/stabilizer: 0.01 to 5%(w/v), preferably 0.1 to 1%(w/v)
Buffer/solution-state stabilizer: 1 to 20% (w/v), preferably 7 to 14% (w/v)

The form of the liposome preparation of the invention is not particularly limited, and the form may be a known form such as a sphere and an ellipse. Moreover, the structures of liposomes are classified into multilamellar vesicles (MLV) and unilamellar vesicles, and a unilamellar vesicle liposome is preferable. The unilamellar vesicle liposome is preferably further subjected to size reduction.

The size reduction method is not particularly limited, but examples thereof include membrane extrusion method, high shear homogenization method and sonication method. Of the methods, membrane extrusion method is preferable.

In the liposome preparation of the invention, when the polyethylene glycol-modified phospholipid is DSPE-$PEG_{2000}$-COOH and/or DSPE-$PEG_{2000}$-$NH_2$, transferrin receptor T12 peptide and/or transferrin is preferably further bonded, and when the polyethylene glycol-modified phospholipid is DSPE-$PEG_{2000}$-COOH, transferrin receptor T12 peptide and/or transferrin is preferably further bonded. The transferrin receptor T12 peptide and/or the transferrin is commercially available from various manufacturers. Examples of the commercial products thereof include BBB-penetrated transferrin receptor (TfR) binding peptide (Cat. No.: HY-P2297A) of MedChemExpress. When being bonded with transferrin receptor T12 peptide and/or transferrin through the COOH group and/or the $NH_2$ group, the polyethylene glycol-modified phospholipid and the transferrin receptor T12 peptide and/or the transferrin are bonded through a peptide bond of -NHCO- and/or -CONH-.

The order of binding transferrin receptor T12 peptide and/or transferrin to DSPE-$PEG_{2000}$-COOH and/or DSPE-$PEG_{2000}$-$NH_2$ is not particularly limited, but for example, transferrin receptor T12 peptide and/or transferrin may be bonded before forming a liposome with DSPE-$PEG_{2000}$-COOH and/or DSPE-$PEG_{2000}$-$NH_2$, and then a liposome may be formed. Alternatively, after a liposome is formed with DSPE-$PEG_{2000}$-COOH and/or DSPE-$PEG_{2000}$-$NH_2$, transferrin receptor T12 peptide and/or transferrin may be bonded. The method for binding transferrin receptor T12 peptide and/or transferrin to DSPE-$PEG_{2000}$-COOH and/or DSPE-$PEG_{2000}$-$NH_2$ is not particularly limited, and a known method may be used for binding.

The properties of the final liposome preparation of the invention are not particularly limited as long as the liposome preparation is stable, is free of contamination of foreign substances which affect the human body and has pH, an osmotic pressure and the like which are close to those of the serum or the body fluid, but examples thereof include those shown below. The liposome preparation may have one or more of the properties and preferably have all thereof. In this regard, the average particle size, the polydispersity index and the zeta potential are values obtained by diluting the preparation five-fold with a buffer solution and measuring with Litesizer 500. Moreover, the deazaflavin concentration in the *in vitro* permeability evaluation is a value measured by the HPLC-UV method. The entrapment efficiency is also calculated from a value measured by the HPLC-UV method.

pH: 6 to 8, preferably 7 to 8

Assay: 55 to 110%, preferably 90 to 110%
Entrapment efficiency of deazaflavin: more than 40%, preferably more than 60.0%
Average particle size: less than 110 nm, preferably less than 100 nm, more preferably 50 nm to less than 100 nm
Polydispersity index: less than 0.30, preferably 0.05 to less than 0.30
Zeta potential: in the range of ±30 mV, preferably in the range of ±20 mV

The liposome preparation of the invention explained above contains a deazaflavin and thus has similar redox (oxidation-reduction) function to those of $NAD^+$ and FAD of the deazaflavin and can activate ATP production in the cells. Accordingly, the liposome preparation of the invention can be used as an activator for ATP production in the cells.
Specifically, the liposome preparation of the invention can activate ATP production and thus can be used as an agent for treating or preventing a disease which can be treated·prevented through activation of ATP production.
Examples of the disease include diabetes, renal failure, obesity, Alzheimer's disease, Parkinson's disease, a neurodegenerative disease following intracerebral hemorrhage·cerebral infarction and depression.
The liposome preparation of the invention is particularly excellent in activating ATP in the mitochondrial complex and is thus preferable for an agent for treating or preventing a brain disease such as Alzheimer's disease, Parkinson's disease, a neurodegenerative disease following intracerebral hemorrhage·infarction and depression, of the diseases. In this regard, the liposome preparation of the invention can pass through the blood-brain barrier and is thus preferable for an agent for treating or·preventing the brain disease.
The capacity range of the liposome preparation of the invention for a mammal including a human is appropriately selected depending on the efficacy, the dosage form and the route of administration of the deazaflavin contained in the liposome preparation of the invention, the properties of the subject (body weight, age, conditions, use of other medications and the like), the decision of the doctor in charge and the like, but the dose of the deazaflavin may be, for example, 1 to 100 mg/day and preferably 10 to 50 mg/day, which may be divided into portions and administered.

## Examples

The invention is explained in detail below referring to Examples, but the invention is not limited to the Examples.

### Example 1

Preparation of Liposome Preparation:
Using the components shown in Table 1 below, a liposome preparation was prepared by solvent injection method and then subjected to size reduction by membrane extrusion method. First, ammonium sulfate, sucrose and histidine as buffers/-solution-state stabilizers and ethanol, water and physiological saline as solvents were added to a round flask and mixed, and thus a buffer solution was prepared. Benzyl alcohol as a solvent, DSPE-$PEG_{2000}$-COOH as a polyethylene glycol-modified phospholipid, HSPC as a surfactant, cholesterol as an emollient/stabilizer and NMN-T as a drug were added to a separate container, and thus a mixture solution was obtained. The mixture solution was injected with a syringe to the buffer solution which was stirred at 65°C. Then, the ethanol was evaporated, and thus a suspension containing a liposome preparation was prepared.

[Table 1]

| | HY8448 |
|---|---|
| NMN-T | 0.05% w/v |
| Benzyl alcohol | 1.00% w/v |
| DSPE-$PEG_{2000}$-COOH | 0.50% w/v |
| HSPC | 1.50% w/v |
| Cholesterol | 0.55% w/v |
| Ammonium sulfate | 0.06% w/v |
| Sucrose | 9.40% w/v |
| Histidine | 0.15% w/v |
| Ethanol | 5.00% v/v |
| Water | Amount resulting in 1 mL |

(continued)

| | HY8448 |
|---|---|
| Appearance of Suspension (before size reduction) | A suspension like a yellow cloud having a small amount of separation and precipitation. |
| pH | 7.55 |

The size of the liposome preparation contained in the suspension containing the liposome preparation was reduced by membrane extrusion method. First, the suspension was allowed to pass through a polycarbonate membrane filter of 100 nm ten times while being maintained at 65°C and then allowed to pass through a polycarbonate membrane filter of 30 nm ten times.

The results of measurement of the pH of thus obtained liposome preparation are also shown in Table 1. Moreover, the appearance of the suspension before and after the size reduction was evaluated in free form. The evaluation is also shown in Table 1.

Test Example 1

Storage Test:

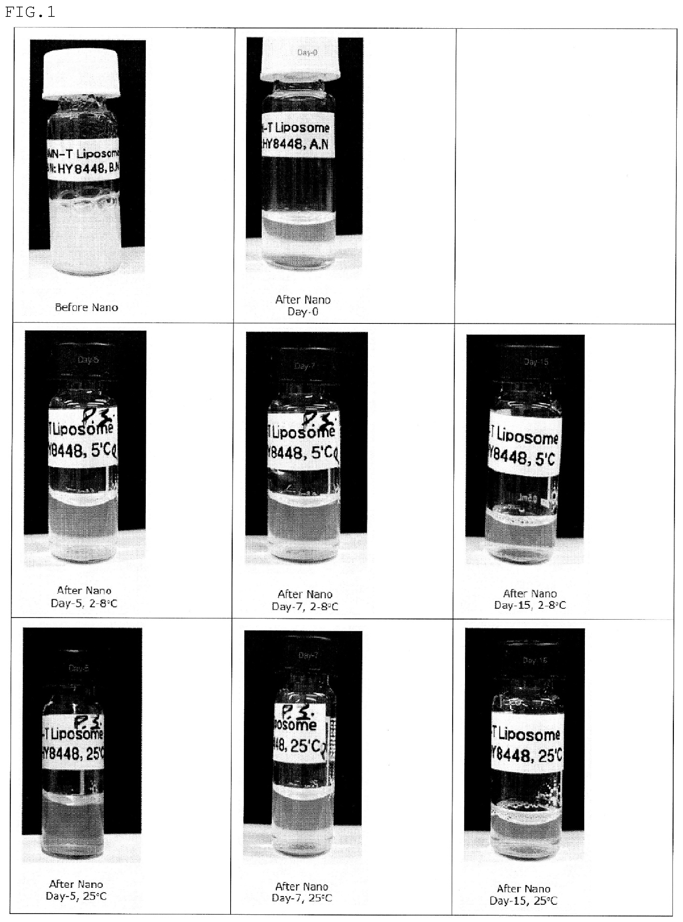

The appearance of the liposome preparation prepared in Example 1 before the size reduction (Nano), after the size reduction and after storage at 2 to 8°C or 25°C for five, seven or 15 days is shown in FIG. 1.

From the results, it was found that HY8448, which is a liposome preparation containing DSPE-PEG$_{2000}$-COOH, is stable also after size reduction.

Example 2

Preparation of Liposome Preparation:

The procedures up to the size reduction were conducted using the components shown in Table 2 below in the same manner as in Example 1, and thus a liposome preparation was prepared.

[Table 2]

| | HY8455 |
|---|---|
| NMN-T | 0.05% w/v |
| Benzyl alcohol | 1.00% w/v |
| DSPE-PEG$_{2000}$-COOH | 0.50% w/v |
| HSPC | 1.50% w/v |
| Cholesterol | 0.55% w/v |
| Ammonium sulfate | 0.06% w/v |
| Sucrose | 9.40% w/v |
| Histidine | 0.15% w/v |
| Ethanol | 5.00% v/v |
| Water | Amount resulting in 1 mL |
| Appearance of Suspension (before size reduction) | A suspension like a yellow cloud having a small amount of precipitation. |
| pH | 7.69 |
| Assay (%) Before Extrusion | 106 |
| Assay (%) After Extrusion | 96.9 |
| Entrapment Efficiency (%) | 68.7 |
| Average Particle Size (nm) | 79.7 |

(continued)

| | HY8455 |
|---|---|
| Polydispersity Index | 0.22 |
| Zeta Potential (mV) | -26.8 |

The results of measurement of the pH, the assays (%) before and after extrusion, the entrapment efficiency (%), the average particle size and the polydispersity index of thus obtained liposome preparation are also shown in Table 2. Moreover, the appearance of the suspension before and after the size reduction was also evaluated in free form. The evaluation is also shown in Table 2.

Here, the total drug analysis (assay (%)) before and after extrusion and the entrapment efficiency (%) are values measured by the HPLC-UV method.

(1) Total Drug Analysis

Agilent 1100HPLC (manufactured by Agilent Technologies) having a diode array detector was used. The drug was separated using an XDB-C18 (4.6 × 50 mm, 1.8 μm) column having (C18: 4.6 × 5 mm) as a precolumn at 50°C. The mobile phases contained 0.1% aqueous FA solution (A) and 0.1% FA acetonitrile solution (B). The flow rate was in a gradient mode (0 minute 5%, five minutes 5%, 10 minutes 80%, 12 minutes 95%, 14 minutes 95% 14.1 minutes 5%, and 15 minutes 4%), and the flow rate was 1.50 mL/min. The injection amount was 5.00 μL. The wavelength of the detector was 328 nm.

(2) Entrapment Efficiency

After 150 μL (or 200 μL) of the preparation sample was injected to a 30 K cut-off filter (Amicon Ultra 0.5 mL centrifuge filter, Ultracel 30K (30 000 Da cut-off), regenerated cellulose 30000 nominal molecular weight limit (NMWL)), the filter was centrifuged at 9700 rpm for 10 minutes at room temperature for separation. By the operation, the free drug passes through the filter while the liposome remains on the filter. The free drug which had passed through the filter was diluted 10-fold with ethanol. The concentration of the free drug in the preparation was determined through analysis by HPLC-UV, and the entrapment efficiency was calculated by the following equation.

Entrapment efficiency (%) = 1 - (free drug concentration/total drug concentration) × 100 [Math. 1]

Moreover, the average particle size, the polydispersity index and the zeta potential are values obtained by diluting the preparation five-fold with the buffer solution and measuring with Litesizer 500 (manufactured by Anton Paar).

Test Example 2

Blood-Brain Barrier Permeability Test:

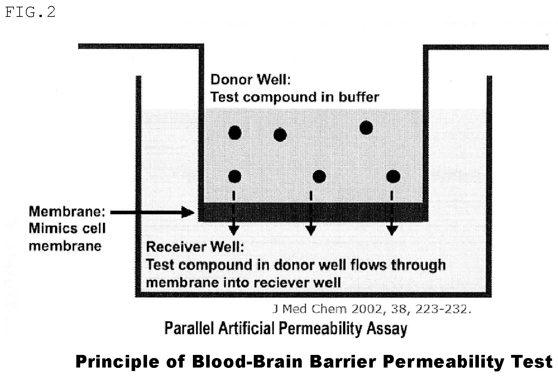

A blood-brain barrier permeability test was conducted using the liposome preparation prepared in Example 2. For the blood-brain barrier permeability test, Parallel Artificial Membrane Permeability Assay-BBB Kit (Creative Bioarray (Cat. No.: DPK-YS003, Lot No.: 022209001YS) was used. The test was conducted following the protocols of Creative Bioarray. As shown in FIG. 2, a donor well was set in an acceptor well (receicer well), and the absorbance of the test compound solution which passed through the membrane that was pre-treated with brain lipid and which moved to the acceptor well was measured to calculate the membrane permeability. The test was conducted at 37°C for 18 hours. For controls of high permeability and low permeability, the UV absorbances at 250 nm and 270 nm were measured with a NanoDrop microvolume spectrophotometer. The NMN-T concentration of the preparation was measured by HPLC-UV. The permeability (Pe) was calculated by the following equation. The results are shown in Table 3.

[Math. 2]

$$Pe = C \times \left(-\ln\left(1 - \frac{OD_A}{OD_E}\right)\right) cm/s$$

In the equation, $OD_A$ is the absorbance (concentration) of the acceptor solution, and $OD_E$ is the absorbance (concentration) of the parallel control. When the incubation period is 18 hours, C is $7.72 \times 10^{-6}$.

[Table 3]

| Sample | Permeation Rate ($\times 10^{-6}$ cm/s) |
|---|---|
| Low Permeability Control | 2.02 |
| High Permeability Control | 3.64 |
| HY8455 | 4.36 |

From the above results, it was found that the liposome preparation obtained in Example 2 has excellent permeability through the blood-brain barrier and that NMN-T (deazaflavin) reaches the inside of the brain.

Example 3

Preparation of Liposome Preparation:

DSPE-$PEG_{2000}$-COOH (10.0 mg, 3.58 μmol) and EDC HCl (1-(3-dimethylaminopropyl)-3-ethylcarbodiimide hydrochloride: 6.87 mg, 35.8 μmol) were dissolved in 10%-THF-containing MES (2-(N-morpholino)ethanesulfonic acid) buffer (0.01 M) pH 6.0 (3 mL), and the solution was stirred at room temperature for two hours. Next, TfR-T12 TFA peptide (4.01 mg, 2.69 μmol: BBB-penetrated transferrin receptor (TfR) binding peptide of MedChemExpress (Cat. No.: HY-P2297A): this is sometimes referred to as TfR-T12 below) was added. The reaction product was gently stirred at room temperature overnight. The change of the buffer solution with 10% ethanol in MilliQ water was conducted using Satorius centrifugal concentrator (MWCO 2000 Da). Four filtration cycles were conducted, and the concentrated reaction mixture was freeze-dried. Thus, DSPE-$PEG_{2000}$-CONH-TfR-T12 was obtained (11 mg, 2.58 μmol, yield: 96%). It was observed by LC/Q-TOF that DSPE-$PEG_{2000}$-CONH-TfR-T12 was obtained.

<Reference Document>

Mu, LM., Bu, YZ., Liu, L. et al. Lipid vesicles containing transferrin receptor binding peptide TfR-T12 and octa-arginine conjugate stearyl-R8 efficiently treat brain glioma along with glioma stem cells. Sci Rep 7, 3487 (2017). https://doi.org/10.1038/s41598-017-03805-7.

The procedures up to the size reduction were conducted using the components shown in Table 4 below including DSPE-$PEG_{2000}$-CONH-TfR-T12 prepared above in the same manner as in Example 1, and thus a liposome preparation was prepared. The results of measurement of the pH, the assays (%) before and after extrusion, the entrapment efficiency (%), the average particle size and the polydispersity index of the obtained liposome preparation are also shown in Table 4. Moreover, the appearance of the suspension before the size reduction was also evaluated in free form. The evaluation is also shown in Table 4.

[Table 4]

| | HY8456 |
|---|---|
| NMN-T | 0.05% w/v |
| Benzyl alcohol | 1.00% w/v |
| DSPE-$PEG_{2000}$-CONH-TfR-T12 | 0.60% w/v |
| DSPE-$PEG_{2000}$-COOH | 0.07% w/v |
| HSPC | 1.50% w/v |
| Cholesterol | 0.55% w/v |
| Ammonium sulfate | 0.06% w/v |
| Sucrose | 9.40% w/v |
| Histidine | 0.15% w/v |
| Ethanol | 5.00% v/v |
| Water | Amount resulting in 1 mL |
| Appearance of Suspension (before size reduction) | A suspension like a yellow cloud having a small amount of precipitation. |

(continued)

| | HY8456 |
|---|---|
| Appearance of Suspension (after size reduction) | A slightly unclear yellow liquid. |
| pH | 7.38 |
| Assay (%) Before Extrusion | 64.5 |
| Assay (%) After Extrusion | 55.2 |
| Entrapment Efficiency (%) | 41.7 |
| Average Particle Size (nm) | 91.6 |
| Polydispersity Index | 0.24 |
| Zeta Potential (mV) | -10.9 |

From the results, it was found that HY8456, which is a liposome preparation containing DSPE-PEG$_{2000}$-CONH-TfR-T12, is stable also after size reduction. Moreover, because TfR-T12 is bonded to the preparation, the permeability through the blood-brain barrier can be superior to that of a preparation to which TfR-T12 is not bonded.

Example 4

Preparation of Liposome Preparation:

The procedures up to the size reduction were conducted in the same manner as in Example 1 except that DSPE-PEG$_{2000}$-NH$_2$ was used instead of DSPE-PEG$_{2000}$-COOH, and thus a liposome preparation was prepared.

Industrial Applicability

Using the liposome preparation of the invention, a deazaflavin can be used for treating or preventing various diseases.

## Claims

1. A liposome preparation comprising a deazaflavin and a polyethylene glycol-modified phospholipid.

2. The liposome preparation according to claim 1, wherein the polyethylene glycol-modified phospholipid is DSPE-PEG$_{2000}$-COOH and/or DSPE-PEG$_{2000}$-NH$_2$.

3. The liposome preparation according to claim 1, wherein the polyethylene glycol-modified phospholipid is DSPE-PEG$_{2000}$-COOH and/or DSPE-PEG$_{2000}$-NH$_2$, and transferrin receptor T12 peptide and/or transferrin is further bonded.

4. The liposome preparation according to any one of claims 1 to 3 which is for activating ATP production in a cell.

5. The liposome preparation according to any one of claims 1 to 3 which is for treating or preventing a brain disease.

FIG.1

Before Nano

After Nano Day-0

After Nano Day-5, 2-8°C

After Nano Day-7, 2-8°C

After Nano Day-15, 2-8°C

After Nano Day-5, 25°C

After Nano Day-7, 25°C

After Nano Day-15, 25°C

FIG.2

Donor Well:
Test compound in buffer
Membrane:
Mimics cell
membrane
Receiver Well:
Test compound in donor well flows through
membrane into reciever well
J Med Chem 2002, 38, 223-232.
Parallel Artificial Permeability Assay

**Principle of Blood-Brain Barrier Permeability Test**

## INTERNATIONAL SEARCH REPORT

International application No. **PCT/JP2024/040391**

**A. CLASSIFICATION OF SUBJECT MATTER**

***A61K 31/519***(2006.01)i; ***A61K 9/1271***(2025.01)i; ***A61K 47/10***(2017.01)i; ***A61K 47/24***(2006.01)i; ***A61P 25/00***(2006.01)i; ***A61P 43/00***(2006.01)i

FI: A61K31/519; A61P25/00; A61P43/00 105; A61K9/1271; A61K47/24; A61K47/10

According to International Patent Classification (IPC) or to both national classification and IPC

**B. FIELDS SEARCHED**

Minimum documentation searched (classification system followed by classification symbols)

A61K31/519; A61K9/1271; A61K47/10; A61K47/24; A61P25/00; A61P43/00

Documentation searched other than minimum documentation to the extent that such documents are included in the fields searched

Published examined utility model applications of Japan 1922-1996
Published unexamined utility model applications of Japan 1971-2025
Registered utility model specifications of Japan 1996-2025
Published registered utility model applications of Japan 1994-2025

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| Y | JP 2019-137673 A (TERA STONE CO., LTD.) 22 August 2019 (2019-08-22) claims, paragraph [0002], examples | 1-5 |
| Y | 小田雄介ら, リポソーム技術を駆使した新規デリバリーシ ステム, 薬剤学, 2010, vol. 70, no. 1, pp. 21-26, (ODA, Yusuke et al., Drug and Gene Delivery System Using Liposome Technology, Journal of Pharmaceutical Science and Technology, Japan) p. 21, left column to p. 22, left column | 1-5 |
| Y | JP 2004-525858 A (THE REGENTS OF THE UNIVERSITY OF CALIFORNIA) 26 August 2004 (2004-08-26) paragraphs [0008]-[0011] | 1-5 |
| A | JP 3-81276 A (FUJIMOTO BROTHERS KK) 05 April 1991 (1991-04-05) entire text | 1-5 |

☐ Further documents are listed in the continuation of Box C.  ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 January 2025** | **21 January 2025** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/040391**

| Patent document cited in search report | Publication date (day/month/year) | Patent family member(s) | Publication date (day/month/year) |
|---|---|---|---|
| JP 2019-137673 A | 22 August 2019 | US 2020/0246340 A1<br>claims, paragraphs [0002], [0003], examples<br>WO 2019/151516 A1<br>EP 3750543 A1<br>KR 10-2020-0024283 A<br>CN 111032051 A | |
| JP 2004-525858 A | 26 August 2004 | US 2002/0054902 A1<br>paragraphs [0015]-[0019], [0040]<br>WO 2001/082900 A1<br>EP 1282403 A1 | |
| JP 3-81276 A | 05 April 1991 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 6717989 B


### Non-patent literature cited in the description


- **MU, LM.** ; **BU, YZ.** ; **LIU, L. et al.** Lipid vesicles containing transferrin receptor binding peptide TfR-T12 and octa-arginine conjugate stearyl-R8 efficiently treat brain glioma along with glioma stem cells.. *Sci Rep*, 2017, vol. 7, 3487, https://doi.org/10.1038/s41598-017-03805-7